# EUROPÄISCHE PATENTSCHRIFT

(11) **EP 2 945 611 B1**
(45) Veröffentlichungstag und Bekanntmachung des Hinweises auf die Patenterteilung: **21.08.2019**
(21) Anmeldenummer: 13798322.7
(22) Anmeldetag: 28.11.2013
(51) Int. Cl.: A61K 8/60, A61K 8/73, A61K 8/81, A61K 8/20, A61Q 19/10

(54) **PEELENDE REINIGUNGSZUBEREITUNG MIT WASSERLÖSLICHEN KRISTALLEN**
EXFOLIATING CLEANSING PREPARATION COMPRISING WATER-SOLUBLE CRYSTALS
COMPOSITION DE NETTOYAGE PAR PELAGE PRÉSENTANT DES CRISTAUX SOLUBLES DANS L'EAU

(30) Priorität: 19.12.2012 DE 102012223743
(43) Veröffentlichungstag der Anmeldung: 25.11.2015
(73) Patentinhaber: Beiersdorf AG, 20253 Hamburg (DE)
(72) Erfinder: SCHORNSTEIN, Ina, 25421 Pinneberg (DE); ARGEMBEAUX, Horst, 21465 Wentorf (DE); SUKOWSKI, Verena, 49201 Dissen (DE)
(86) Internationale Anmeldenummer: PCT/EP2013/074979
(87) Internationale Veröffentlichungsnummer: WO 2014/095295

(56) Entgegenhaltungen:
- EP-A2- 0 584 877
- WO-A1-2008/023145
- WO-A1-2008/034549
- WO-A1-2008/047148
- WO-A2-2012/167903

## Beschreibung

Die vorliegende Erfindung beschreibt kosmetische Reinigungszubereitungen, welche neben Tensiden wasserlösliche Bestandteile in gelöster und kristalliner Form enthalten, die durch polymere Strukturanten stabil in der Zubereitung verteilt sind.

Die Reinigung des Körpers ist von alters her ein Bedürfnis des Menschen. In der Art und Weise und Häufigkeit der Reinigung hat es über die Jahrhunderte große Veränderungen gegeben. Heutzutage ist es für viele Menschen üblich, dass zumindest einmal am Tag eine Ganzkörperreinigung in Form eines Duschbades oder Vollbades erfolgt. Zu diesem Zweck verwandte Dusch-und Badezubereitungen sind an sich bekannt. Jedoch gibt es immer neue Wünsche und Bedürfnisse der Verbraucher, denen die kosmetische Industrie Rechnung trägt, indem immer wieder neue, verbesserte und veränderte Zubereitungen zur Verfügung gestellt werden.

Zur tiefer gehenden Hauterfrischung und -reinigung gibt es Reinigungsmittel, die einen Peeling-Effekt aufweisen.

Unter Peeling versteht man eine kosmetische oder dermatologische Behandlung, bei der die oberflächlichen Schichten der Haut abgetragen werden. Bei einem oberflächlichen Peeling wird die oberste Hornschicht der Haut mechanisch oder chemisch entfernt. Bekannt sind auch tiefer gehende Peelingverfahren, bei denen die gesamte Hornschicht oder auch die Haut bis zur Kollagenschicht abgetragen wird.

Unterschieden werden chemische und mechanische Verfahren. Beim chemischen Peeling werden Fruchtsäuren, Lipo-Hydroxy-Säure, Trichloressigsäuren, Phenolverbindungen und Vitamin A-Säure eingesetzt. Die beiden erstgenannten Verbindungsklassen sind für das oberflächliche Peeling geeignet.

Im Stand der Technik gibt es eine Vielzahl von Offenbarungen zu Zubereitungen mit einem Peelingeffekt.
WO 2012/167903 A2 sei beispielhaft genannt. Es werden Zubereitungen beschrieben, die partikuläres Polyglyceryl-10-stearat enthalten, das als Peelingmittel wirkt.

Im Dokument EP 584877 A2 werden viskoelastische Zubereitungen in Gelform offenbart, die Tenside und einen quervernetzten Verdicker in Gelform, sowie wahlweise auch abrasiv wirkende Komponenten, enthalten.

Beim mechanischen Peeling können verschiedene Substanzen zum Einsatz kommen, von denen hier einige aufgeführt werden:
Aluminiumoxid-Mikropartikel, Polydimethylsilikonharz-Mikropartikel, harte Bürsten, Mikrofasertücher, Tonerde, Sand, Kaffeesatz, Kunststoffpartikel, zerstoßene oder gemahlene Kerne von Aprikosen oder Pfirsichen, Wachs.

Auch geeignet sind Zucker oder Salz. Da diese sich in Wasser lösen, werden sie meist in wasserfreien Zubereitungen eingesetzt. Sie können aber auch in wässrigen Systemen in so hohen Konzentrationen eingesetzt werden, dass Zucker- oder Salzkristalle vorliegen. Derartige Kristalle entfalten eine gute Schälwirkung.

Um diese Kristalle homogen verteilt in der wässrigen Zubereitung zu halten, ohne dass sich nennenswerte Mengen am Boden absetzen, eignen sich Tensidsysteme, die Strukturanten enthalten.

Im Stand der Technik gibt es bereits Dokumente, die die homogene Verteilung von Partikeln in Tensidsystemen beschreiben. Diese Systeme weisen jedoch einen charakteristischen Aufbau auf und werden im Englischen als "Structured Surfactant Systems" bezeichnet, im Deutschen wird im Folgenden die Bezeichnung strukturierte Tensidsysteme verwendet. Diese Systeme zeichnen sich durch eine lamellare Phase (auch Mesophase oder G-Phase genannt) und meist eine eingeschobene wässrige Phase aus. Die lamellare Phase ist durch Doppelschichten (im Englischen wird der Begriff Bilayer verwendet) von Tensiden gekennzeichnet, bei denen die hydrophoben Enden der Tenside in der Regel nach innen zeigen und die hydrophilen Enden nach außen. Die Doppelschichten liegen nebeneinander mit einer parallelen oder konzentrischen Ausrichtung, sie können durch wässrige Schichten getrennt sein. Eine besondere Eigenschaft dieser Systeme ist es, dass sie in der Lage sind suspendierte Partikel in Lösung zu halten, aber auch ein Ausgießen oder Fließen zu erlauben.

Die meisten strukturierten Tensidsysteme enthalten Elektrolyte, Tenside und Wasser um feste Partikel in Schwebe zu halten.

Es gibt im Stand der Technik Dokumente, die strukturierte Tensidsysteme für den Einsatz in der Kosmetik beschreiben. Das Dokument EP 1203068 offenbart ein Tensidsystem mit erweiterten lamellaren Phasen, welches gebildet wird aus Tensiden, Wasser und Strukturanten, die ein wasserlösliches Kohlenhydrat oder auch ein Elektrolyt sein können. Ein derartiges Tensidsystem ist in der Lage Partikel stabil zu suspendieren.

Das Dokument WO 2008/023145 offenbart strukturierte Tensidsysteme, die zur Hautabschuppung verwendet werden. Die Hautabschuppung erfolgt durch feste Zuckerpartikel, die in eine gesättigte Lösung des Zuckers gegeben werden zusammen mit ausreichend Tensid. Als zusätzliche strukturgebende Substanzen werden Elektrolyte offenbart.

Die vor genannten Schriften offenbaren strukturierte Tensidsysteme, die Partikel enthalten. In der Herstellung und im Zusammenspiel der einzelnen Komponenten sind strukturierte Systeme anspruchsvoll und der Umgang mit derartigen Systemen in der Kosmetik ist nicht frei von Rückschlägen.

Die Aufgabe, die der vorliegenden Erfindung zugrunde liegt, besteht darin, kosmetische Reinigungszubereitungen mit stabil suspendierten Partikeln zur Verfügung zu stellen, deren Zusammensetzung einfach zu formulierende Komponenten enthält und deren Herstellung auf einfache Weise erfolgt.

Weiterhin ist es die Aufgabe der vorliegenden Erfindung, Zusammensetzungen zur Verfügung zu stellen, die einen Peelingeffekt durch einfache, natürliche Komponenten ermöglichen.

Ebenso ist es die Aufgabe der vorliegenden Erfindung, dass bei der Anwendung der erfindungsgemäßen Zubereitungen keine oder praktisch keine Rückstände auf Waschtischen oder Anwendungseinrichtungen zu hinterlassen werden.

Weiterhin ist es Aufgabe der vorliegenden Erfindung, dass die Substanzen mit Peelingeffekt, insbesondere Kohlenhydratkristalle, homogen in der Zubereitung verteilt sind und auch bleiben.

Die Lösung aller dieser Aufgaben liegt in der Bereitstellung von kosmetischen Reinigungszubereitungen, die Tenside, wasserlösliche Bestandteile in gelöster und ungelöster Form und polymere Strukturanten enthalten. Die wasserlöslichen Bestandteile in ungelöster Form liegen ganz oder zumindest zum überwiegenden Teil als Kristalle vor.

Erfindungsgemäß sind Zubereitungen, die kein strukturiertes Tensidsystem enthalten.

Erfindungsgemäß ist, dass in den erfindungsgemäßen Zubereitungen anionische und/oder amphotere und/oder kationische und/oder nichtionische Tenside enthalten sind.

Der Gehalt an Tensiden in den erfindungsgemäßen Zubereitungen beträgt 0,1 bis
10 Gew.-%, bevorzugt 0,5 bis 8 Gew.-%, bezogen auf das Gesamtgewicht der Zubereitung.

Erfindungsgemäß sind auch Zubereitungen mit einem Tensidgehalt > 10 bis 15 Gew.-%, bezogen auf das Gesamtgewicht der Zubereitung, die kein strukturiertes Tensidsystem aufweisen.

Erfindungsgemäß sind kosmetische Reinigungszubereitungen mit wasserlöslichen Bestandteilen in gelöster und kristalliner Form, wenn es sich bei diesen um Kohlehydrate handelt.

Die erfindungsgemäß einsetzbaren Kohlenhydrate können beispielsweise Saccharide sein, wie Mono-, Di-, Oligo- oder Polysaccharide. Mono- oder Disaccharide können beispielsweise Saccharose, Glukose oder Fruktose sein, wobei Saccharose bevorzugt ist.

Der Gesamtkohlenhydratgehalt beträgt 40 bis 90 % Gew.-%, bevorzugt 50 bis 80 Gew.-%, besonders bevorzugt 60 bis 75 Gew.-% bezogen auf das Gesamtgewicht der Zubereitung.

Die Salze können beispielsweise Alkalisalze sein, wie beispielsweise Natriumchlorid, Kaliumchlorid, Natriumsulfat und/oder Kaliumsulfat, wobei Natriumchlorid bevorzugt ist. Die Salze können ebenfalls Erdalkalisalze sein, wie beispielsweise Magnesiumchlorid und/oder Magnesiumsulfat.

Der Gesamtgehalt an Salzen beträgt 8,0 bis 80 Gew.-%, bevorzugt 20 bis 70 Gew.-%, besonders bevorzugt 40 bis 65 Gew.-%, bezogen auf das Gesamtgewicht der Zubereitung.

Erfindungsgemäß sind kosmetische Reinigungszubereitungen mit polymeren Strukturanten, wobei die Strukturanten vernetzte Copolymere sind, wobei die vernetzten Copolymere Copolymere aus Vinylpyrrolidon und Acrylsäure sind.

Der Gehalt an polymeren Strukturanten beträgt 0,05 bis 3,0 %, bevorzugt 0,1 bis
2,0 Gew.-%, bezogen auf das Gesamtgewicht der Zubereitung.

Erfindungsgemäß vorteilhaft ist auch der Einsatz von Xanthangummi mit einem Gehalt von 0,05 bis 0,8 Gew.-%, bevorzugt 0,1 bis 0,5 Gew.-%.

Erfindungsgemäß sind ebenfalls kosmetische Reinigungszubereitungen mit
- Tensiden,
- Zuckern in gelöster und kristalliner Form und
- als Strukturanten vernetzte Copolymere aus Vinylpyrrolidon und Acrylsäure,
- aufweisend eine dynamische Viskosität < 80.000 mPa s bei 25°C,
- wobei die Verteilung der Zuckerkristalle in der Zubereitung homogen und stabil ist.

Ebenso erfindungsgemäß ist die Verwendung von polymeren Strukturanten zur Hemmung der Sedimentationsneigung von Kohlenhydratkristallen in Kohlenhydrat-gesättigten, tensidhaltigen kosmetischen Reinigungszubereitungen.

Erfindungsgemäß vorteilhaft ist die Verwendung von polymeren Strukturanten zur Hemmung der Sedimentationsneigung von Zuckerkristallen in Zucker-gesättigten, tensidhaltigen kosmetischen Reinigungszubereitungen.

In der vorliegenden Offenbarung werden die Begriffe Strukturant, Struktur-Bildner, Gelbildner oder Struktur-gebende Substanz synonym verwendet.

Die in der vorliegenden Erfindung verwendeten Strukturanten erlauben es erfindungsgemäße Zubereitungen zur Verfügung zu stellen, die Partikel enthalten, die homogen suspendiert sind und auch bleiben. Es handelt sich um Partikel mit Peelingeffekt, wobei es sich um Kohlenhydratkristalle handelt. Die erfindungsgemäßen Strukturanten sind vernetzte Copolymere, wobei die vernetzten Copolymere Copolymere aus Vinylpyrrolidon und Acrylsäure sind.

Die Strukturanten haben einen Effekt auf die stabile Suspendierung von Partikel. Dies wird belegt durch die Versuche, die in Beispiel 4 (siehe hinten) beschrieben sind.

Die Tensidgehalte in den erfindungsgemäßen Zubereitungen liegen im Bereich von 0,1 bis 10 Gew.-%. Dies sind Bereiche, in denen sich keine strukturierten Tensidsysteme ausbilden. Erfindungsgemäß sind jedoch auch Zubereitungen mit einem Tensidgehalt von > 10 bis 15 Gew.-%, die kein strukturiertes Tensidsystem ausbilden.

Die Viskositätswerte, die in dieser Offenbarung angeführt werden, sind mit dem Rheomat R123 der Gesellschaft ProRheo bei 25°C gemessen worden. Bei der Messung mit dem Rheomat R123 wird der Rotor des Gerätes blasenfrei bis zur Markierung in die Probe eingetaucht. Für die Messungen wurde der Messkörper 2 verwendet. Weitergehende Informationen zum Rheomat R 123 sind im Internet veröffentlicht, siehe
http://www.prorheo.de/fileadmin/user upload/pdfs/R123.pdf und
http://www.prorheo.de/fileadmin/user upload/pdfs/Bedienung R123 d.pdf.

Um Partikel in der Schwebe zu halten ist neben einer angemessenen Viskosität auch die Ausbildung einer Fließgrenze notwendig.

Als Fließgrenze wird die kritische Schubspannung der Fließkurve angesehen. Sie kann erfindungsgemäß wie folgt ermittelt werden:
Gemessen wird die Fließkurve auf einem schubspannungsgesteuerten Rheometer bei 25°C ± 1 °C mit 20 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Eine Fließgrenze bildet sich aus, wenn die Zubereitung neben viskosen Eigenschaften auch einen angemessenen Anteil viskoelastischer Eigenschaften hat. Den Anteil der viskosen und viskoelastischen Eigenschaften kann man mit Frequenzmessungstests bestimmen. Derartige Messungen wurden beispielhaft durchgeführt. Die Abbildungen in Beispiel 4 zeigen die Ergebnisse von Frequenzmessungstests zur Ermittlung des Verhältnisses von elastischen zu viskosen Eigenschaften von einer beispielhaften erfindungsgemäßen Zubereitung. Vermessen wurde Beispiel 1 aus der ersten Serie. Die Messungen wurden mit dem Gerät ARES 5 durchgeführt, bei einer Peltiertemperierung von 25°C. Das Messsystem hatte einen Platte-Platte Durchmesser von 50 mm und einen Spalt von 1mm. Ein Programm von 0,1 -> 100 rad/s wurde verwendet. Bestimmt wurden die elastischen Anteile, beschrieben durch das Speichermodul G', und die viskosen Anteile, beschrieben durch das Verlustmodul G", in der zu vermessenden Probe. Das Verhältnis von G" zu G' ergibt den jeweiligen tan δ Wert. Tan δ Werte unter 1 zeigen an, dass in den vermessenen Zubereitungen die elastischen Eigenschaften überwiegen, ein tan δ Wert von 1 entspricht dem Gelpunkt und tan δ Werte oberhalb von 1 sind ein Zeichen dafür, dass die viskosen Eigenschaften überwiegen.

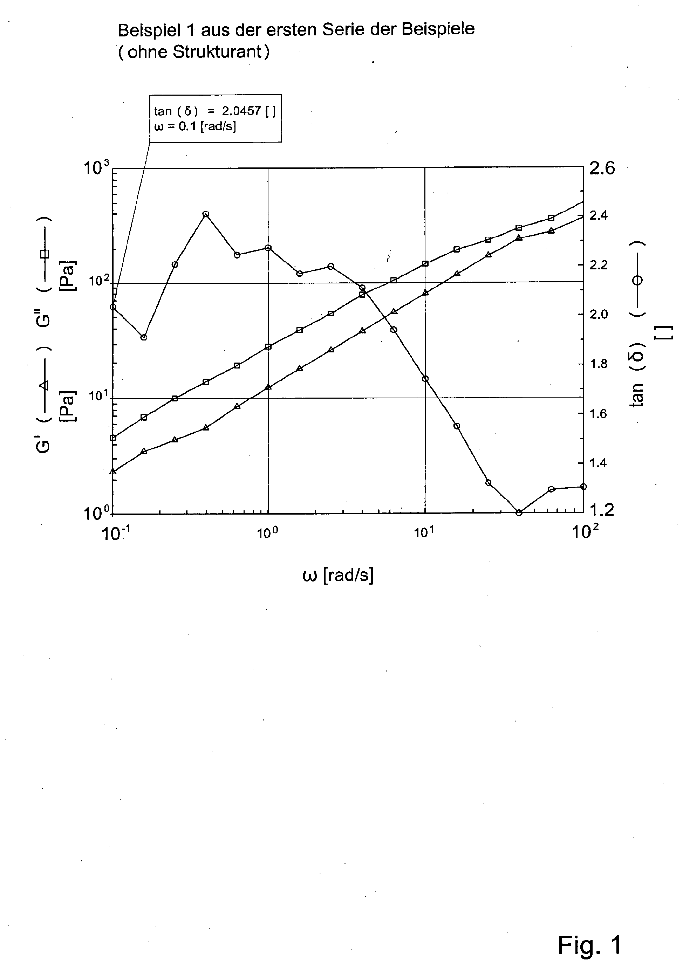

In den Abbildungen (Fig. 1 bis 4) sind die Ergebnisse von Frequenzmessungen zur Bestimmung des Verhältnisses von viskosem zu elastischem Anteil in einer beispielhaften Zubereitung mit und ohne polymerem Strukturant (=Gelbildner) dargestellt.

Die erste Abbildung (Fig. 1) zeigt die Messergebnisse einer Probe ohne polymeren Strukturant (=Gelbildner). Die G"-Werte sind größer als die G'-Werte, d.h. der viskose Anteil in dieser Zubereitung überwiegt. Die tan δ Werte liegen über 1.

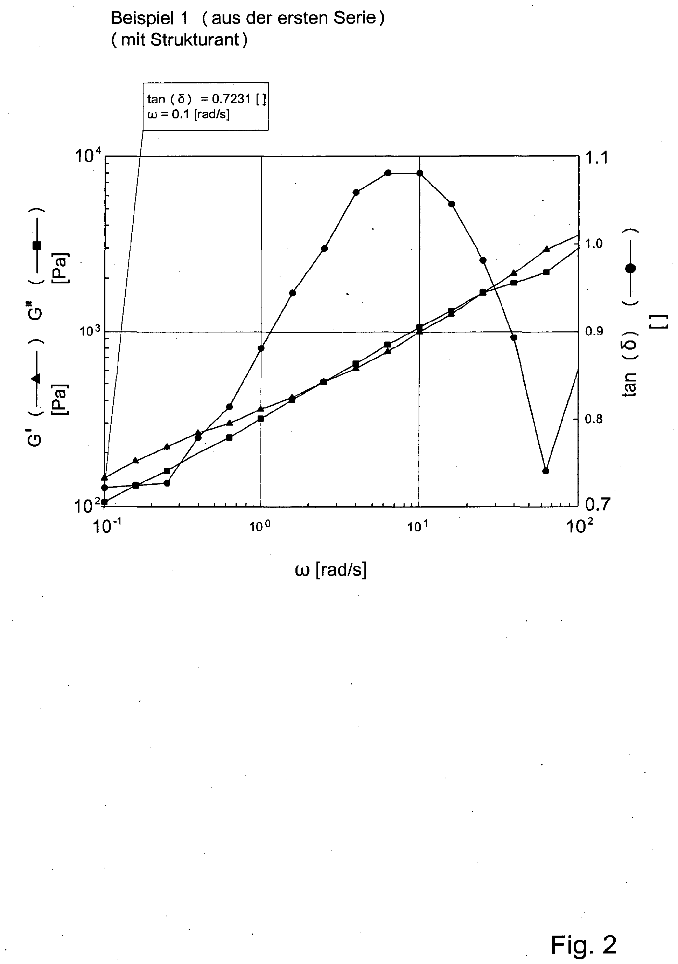

Die zweite Abbildung (Fig. 2) zeigt die Ergebnisse für eine erfindungsgemäße Probe, die einen polymeren Strukturanten (=Gelbildner) enthält. Die erste Probe und die zweite Probe unterscheiden sich durch die Ab- bzw. Anwesenheit des Strukturanten. Die Messergebnisse für die zweite Probe zeigen, dass der elastische Anteil in zwei Frequenzbereichen größer ist als der viskose, dies führt dazu, dass die tan δ Werte fast immer unter 1 liegen.

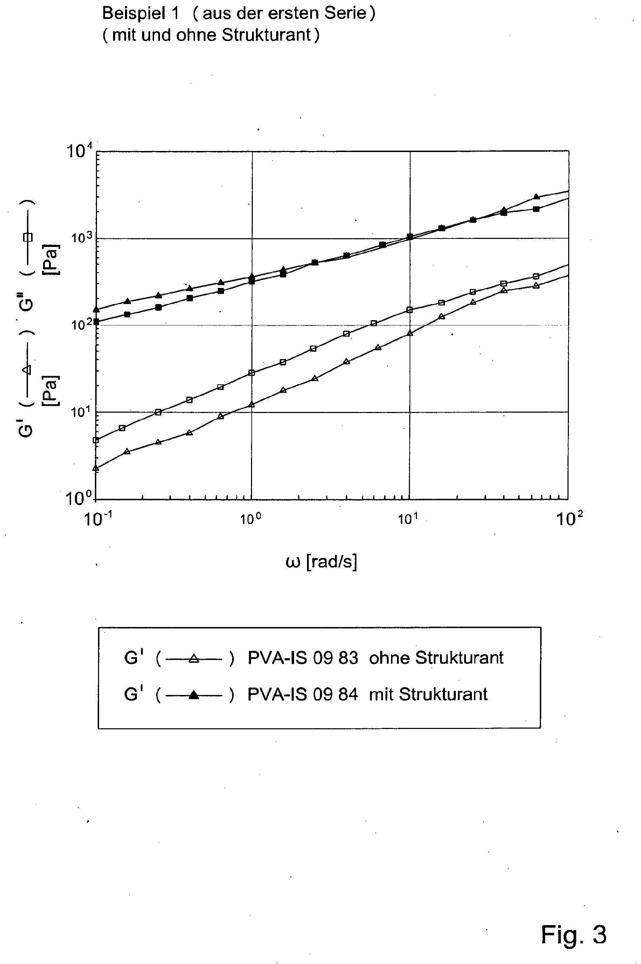

Die dritte Abbildung (Fig. 3) vereinigt die Ergebnisse der ersten zwei Abbildungen hinsichtlich der Werte zu G'- und G". Bei der Betrachtung der G'-Kurven wird deutlich, dass in der Probe ohne polymeren Strukturanten (=Gelbildner) deutlich geringere Werte gemessen werden als in der Probe mit polymerem Strukturanten. Das bedeutet, dass der Zusatz des polymeren Strukuranten zur Erhöhung der elastischen Eigenschaften führt.

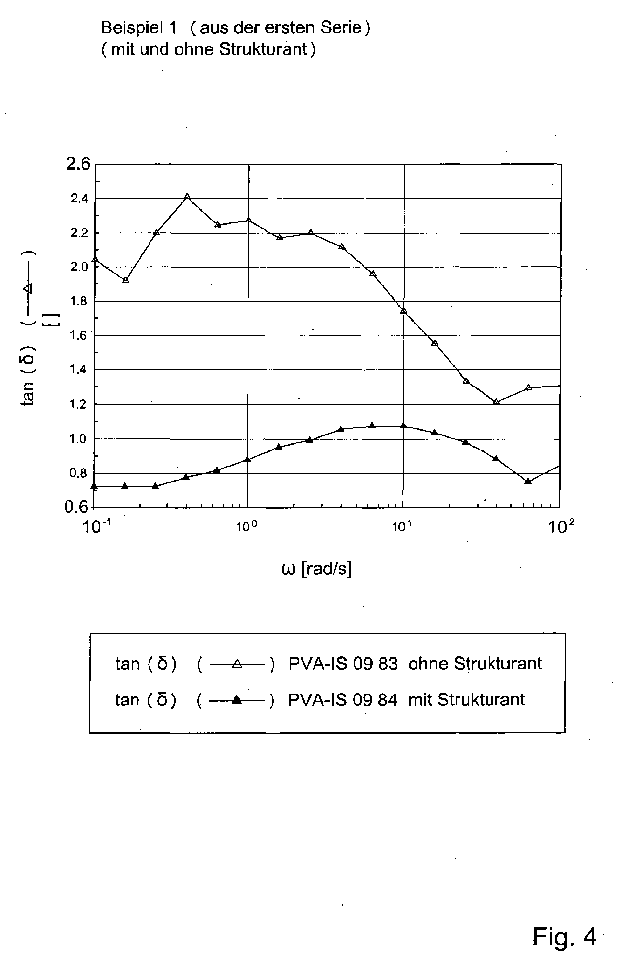

Die vierte Abbildung (Fig. 4) vereinigt die tan δ Werte aus den ersten zwei Abbildungen in einer Abbildung. Die tan δ Werte für die Probe mit polymerem Strukturant (=Gelbildner) liegen weitgehend unter 1. Dies bedeutet, dass die elastischen Anteile überwiegen und das wiederum belegt eine Zunahme an Struktur in der Probe. Dies bewirkt, dass in den erfindungsgemäßen Zubereitungen Zuckerkristalle in Schwebe gehalten werden.

Die *Tenside,* die in den erfindungsgemäßen Zubereitungen Verwendung finden, können anionische und/oder amphotere und/oder kationische und/oder nichtionische Tenside sein.

### Vorteilhaft zu verwendende anionische Tenside sind

Acylaminosäuren (und deren Salze), wie
1. Acylglutamate, beispielsweise Natriumacylglutamat, Natrium Cocoylglutamat, Di-TEApalmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat, Stearinsäure/-salz, Palmitinsäure/-salz,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,
Phosphorsäureester und Salze, wie beispielsweise DEA-Oleth-10-Phosphat und Dilaureth-Phosphat,
Sulfonsäuren und Salze, wie
1. Acyl-isethionate, z.B. Natrium-/ Ammoniumcocoyl-isethionat, Natrium Lauryl Methylisethionate,
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat mit unterschiedlichen Ethoxylierungsgraden und deren Gemische, beispielsweise Natrium-, Ammonium-, Magnesium-, MIPA-, TIPA-Laureth-X-sulfat, Natriummyreth-X-sulfat und Natrium C12-13-Pareth-X-sulfat, mit X = 1-5 Ethoxygruppen.
2. Alkylsulfate, beispielsweise Natrium-, Ammonium- und TEA-Laurylsulfat, Natrium-Ammonium- und TEA-Cocosulfat.

Vorteilhaft zu verwendende kationische Tenside sind
1. Alkylamine,
2. Alkylimidazole,ethoxylierte Amine
3. Quaternäre Tenside, beispielsweise Cetyl Trimethylammoniumhalogenid.
4. Esterquats, beispielsweise Dicocoylethyl Hydroxyethylmoium Methosulfate und
5. Amidquats, beispielsweise Palmitamidopropyltrimonium Chlorid.

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betaine, Cocoamidopropyl Betaine,
4. Sultaine, beispielsweise Lauryl Hydroxy Sultaine.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

*Partikel* in Sinne der vorliegenden Erfindung sind feste Strukturen in Form von Kristallen, die eine Peelingwirkung hervorrufen. Die Peelingwirkung wird durch den ungelösten Anteil an wasserlöslichen Bestandteilen, der in kristalliner Form vorliegen kann, nämlich durch Kristalle von Kohlenhydraten, bevorzugt durch Kristalle von Zuckern, hervorgerufen. Dies wird erreicht durch eine so hohe Zuckerkonzentration, dass sich nicht alle Zuckermoleküle in der erfindungsgemäßen Zubereitung lösen können, sondern in kristalliner Form vorliegen. Dies bedeutet, dass Zuckerkristalle in der Reinigungszubereitung vorliegen. Die Peelingsubstanzen in Form von Zuckerkristallen kommen bei Anwendung während des Dusch- oder Waschvorgangs mit Wasser in Kontakt, beispielsweise beim Abduschen oder Abwaschen des Schaums und der Reinigungszubereitung am Ende des Reinigungsvorgangs. Hierdurch ist ein Auflösen der Zuckerkristalle gewährleistet, was zu einem abnehmenden Peelingeffekt führt. Es bleiben keine oder praktisch keine Rückstände der Peelingsubstanzen in Waschbecken oder Behandlungsvorrichtungen zurück. Praktisch keine Rückstände bedeutet, dass in einzelnen wenigen Fällen äußerst geringe Spuren der Reinigungszubereitung nach dem Waschvorgang auf Waschtischen oder Anwendungseinrichtungen sichtbar sind, die jedoch auf einfache Weise durch Abspülen oder Wegwischen entfernbar sind.

Zusätzliche Peelingmittel sind aufgrund der erfindungsgemäßen Ausführungen überflüssig. Selbstverständlich ist aber eine Zugabe zusätzlicher Peelingmittel möglich. Die Peelingmittel werden dann vorteilhaft gewählt aus der Gruppe Polyethylen, Tonerde, Sand, Kunststoffpartikel, zerstoßene oder zermahlene Kerne von Walnusschalen, Aprikosen-, Pfirsich- und/oder Mandelkernen.

Um den erfindungsgemäßen Zubereitungen rückfettende Eigenschaften zu verleihen können beispielsweise *Öle* in die erfindungsgemäßen Zubereitungen eingearbeitet werden.

Die Öle können ausgewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat,Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether *(Cetiol OE)* und/oder Dicaprylylcarbonat, beispielsweise das unter der Handelsbezeichnung *Cetiol CC* erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C12-13-Alkyllactat, Di-C12-13-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C12-15-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Hallstar AB)* und/oder Diethylhexylnaphthalat *(Hallbrite TQ oder Corapan TQ von H&R).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Bevorzugt sind unpolare Öle, wie beispielsweise verzweigte oder unverzweigte Kohlenwasserstoffe und -wachse, insbesondere Mineralöl, Vaseline (Petrolatum), Paraffinöl, Squalan und Squalen, Polyolefine, hydrogenierte Polyisobutene und Isohexadekan.

Erfindungsgemäße Zubereitungen können ein oder mehrere *Hydrocolloide,* enthalten, die wie polymere Strukturanten im Sinne der Erfindung wirken können. Diese Hydrocolloide können vorteilhaft gewählt werden aus der Gruppe der Gummen, Polysaccharide, Cellulosederivate, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi ausgewählt werden; besonders vorteilhaft ist Xanthangummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können auch Kieselsäuregele verwendet werden.

Vorteilhaft sind außerdem Taurate, z.B. Ammonium Acryloyldimethyltaurat / VP Copolymer.

Unter den Polyacrylaten befinden sich z.B. Carbopol Typen der Firma Lubrizol (Carbopol 980, 981, 1382, 5984, 2984, ETD 2001, ETD 2020, ETD 2050, Ultrez-10 oder Pemulen TR1 & TR2).

Unter den Polymeren befinden sich z.B. Polyacrylamide (Seppigel 305), Polyvinylalkohole, PVP, PVP / VA Copolymere, Polyglycole.

Erfindungsgemäße Zubereitungen können einen oder mehrere *Emulgatoren* enthalten. Bevorzugte Emulgatoren sind W/O-Emulgatoren.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter oder unverzweigter Alkancarbonsäuren einer Kettenlänge von 8 bis 24, insbesondere 12 bis 20 Kohlenstoffatomen, Diglycerinester gesättigter und/oder ungesättigter, verzweigter oder unverzweigter Alkancarbonsäuren einer Kettenlänge von 8 bis 24, insbesondere 12 bis 20 Kohlenstoffatomen, Monoglycerinether gesättigter und/oder ungesättigter, verzweigter oder unverzweigter Alkohole einer Kettenlänge von 8 bis 24, insbesondere 12 bis 20 Kohlenstoffatomen, Diglycerinether gesättigter und/oder ungesättigter, verzweigter oder unverzweigter Alkohole einer Kettenlänge von 8 bis 24, insbesondere 12 bis 20 Kohlenstoffatomen, Polypropylenglykolester gesättigter und/oder ungesättigter, verzweigter oder unverzweigter Alkancarbonsäuren einer Kettenlänge von 8 bis 24, insbesondere 12 bis 20 Kohlenstoffatomen, Polyglycerylester gesättigter und/oder ungesättigter, verzweigter oder unverzweigter Alkancarbonsäuren einer Kettenlänge von 8 bis 24, insbesondere 12 bis 20 Kohlenstoffatomen, Sorbitanester gesättigter und/oder ungesättigter, verzweigter oder unverzweigter Alkancarbonsäuren einer Kettenlänge von 8 bis 24, insbesondere 12 bis 20 Kohlenstoffatomen Lanolinalkohol.

Bevorzugte W/O-Emulgatoren sind verzweigte oder unverzweigte, gesättigte oder ungesättigte Fettsäuren mit 12 bis 26 Kohlenstoffatomen, Polyglyceryl-3 Diisostearat, Polyglyceryl-4 Isostearat, Polyglyceryl-2 Dipolyhydroxystearat, Cetyl PEG/PPG-10-1-Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat.

Besonders bevorzugte W/O-Emulgatoren sind ethoxylierte Fettalkohole, insbesondere Laureth-2.

Erfindungsgemäß sind auch Substanzen, die üblicherweise eingesetzt werden, um den pH-Wert der erfindungsgemäßen Zubereitungen einzustellen und stabil zu halten. Vorteilhaft können diese Substanzen ausgewählt werden aus der Gruppe Zitronensäure und Milchsäure und/oder aus der Gruppe Aminomethylpropanol, Natronlauge, Kalilauge und Triethanolamin.

Optional können, wenn erforderlich, die in der Kosmetik üblichen Zusatz- und Hilfsstoffe in die Zubereitungen eingearbeitet werden, wie z.B. Konservieungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfums, weichmachende Substanzen, anfeuchtende und/oder feuchthaltende Substanzen, Antioxidantien, UV-Filtersubstanzen, Sensorikadditive, Filmbildner, Wirkstoffe oder andere übliche Bestandteile einer kosmetischen Formulierung wie Alkohole, Polyole, Schaumstabilisatoren, Elektrolyte, organische Lösungsmittel oder Silikonderivate.

Die erfindungsgemäßen Zubereitungen zeichnen sich durch eine einfache und sichere Herstellung aus. Die Peelingpartikel sind homogen in der Zubereitung verteilt und bleiben es auch. Weiterhin lassen sich die Zubereitungen problemlos aus den entsprechenden Verpackungen entnehmen. Sie weisen ein entsprechend gutes Fließverhalten auf.

### Beispiele

**Erste Serie an Beispielen (wird auf der nächsten Seite fortgesetzt):**

| **Ansatznummer** | **1** | **2** | **3** | **4** | **5** | **6** | **7** | **8** |
|---|---|---|---|---|---|---|---|---|
| **INCI** | **m [%]** | **m [%]** | **m [%]** | **m [%]** | **m [%]** | **m [%]** | **m [%]** | **m [%]** |
| Aqua | 13,64 | 16,14 | 15,64 | 15,14 | 23,21 | 25,22 | 25,32 | 27,82 |
| Paraffinum Liquidum | 5,00 | 5,00 | 5,00 | 5,00 | | | | |
| Aqua + Sodium Hydroxide (45%) | | | | | | | | |
| Sodium Chloride | 1,50 | 2,00 | 2,50 | 3,00 | | | | |
| Sodium Myreth Sulfate (71%) | 7,70 | 7,70 | 7,70 | 7,70 | | | | |
| Sodium Laureth Sulfate (70%) | | | | | | | | |
| Laureth-2 (99,85%) | 1,20 | 1,20 | 1,20 | 1,20 | | | | |
| Lauryl Glucoside (51,5%) | | | | | | | | |
| Coco-Caprylate/Caprate | | | | | 2,50 | 2,50 | 2,50 | |
| Cocam idopropyl Betaine + Glycerin (34%) | | | | | | | | |
| Decyl Glucoside (53%) | 2,81 | 2,81 | 2,81 | 2,81 | | | | |
| Aminomethyl Propanol (95%) | 0,00 | | | | | | | |
| Coco-Glucoside (52%) | | | | | 5,33 | 2,26 | 2,26 | 2,26 |
| Parfum | 0,15 | 0,15 | 0,15 | 0,15 | | | | |
| Coco-Glucoside + Glyceryl Oleate (70%) | | | | | 0,68 | 0,91 | 0,91 | 0,91 |
| Disodium Cocoyl Glutamate (25%) | | | | | | | | |
| Acrylic Acid/VP Crosspolymer | 1,00 | 1,00 | 1,00 | 1,00 | 1,20 | 1,00 | 0,90 | 0,90 |
| Ammonium Lauryl Sulfate (30%) | | | | | | | | |
| Sucrose | 67,00 | 64,00 | 64,00 | 64,00 | 64,00 | 64,00 | 64,00 | 64,00 |
| Parfum | | | | | | | | |
| Sodium Coco-Sulfate (92,5%) | | | | | 3,08 | 4,11 | 4,11 | 4,11 |
| **Summen**: | **100,00** | **100,00** | **100,00** | **100,00** | **100,00** | **100,00** | **100,00** | **100,00** |

Die Werte in Klammern hinter den Substanznamen beziehen sich auf die Aktivgehalte.

**Erste Serie an Beispielen (Fortsetzung der vorangehenden Seite):**

| **Ansatznummer** | **9** | **10** | **11** | **12** | **13** | **14** | **15** | **16** | **17** |
|---|---|---|---|---|---|---|---|---|---|
| **INCI** | **m [%]** | **m [%]** | **m [%]** | **m [%]** | **m [%]** | **m [%]** | **m [%]** | **m [%]** | **m [%]** |
| Aqua | 9,36 | 16,07 | 15,67 | 22,67 | 16,74 | 16,84 | 16,35 | 16,45 | 22,97 |
| Paraffinum Liquidum | | 5,00 | 5,00 | | 5,00 | 5,00 | 5,00 | 5,00 | |
| Aqua + Sodium Hydroxide (45%) | 0,58 | | | | | | | | |
| Sodium Chloride | | 1,50 | | | 1,50 | 1,50 | | | |
| Sodium Myreth Sulfate (71%) | | 7,70 | | | 7,70 | 7,70 | | | |
| Sodium Laureth Sulfate (70%) | | | 5,71 | | | | 5,71 | 5,71 | |
| Laureth-2 (99,85%) | | 1,20 | | | 1,20 | 1,20 | | | |
| Lauryl Glucoside (51,5%) | 5,48 | | | | | | | | |
| Coco-Caprylate/Caprate | 3,00 | | | 5,00 | | | | | 5,00 |
| Cocam idopropyl Betaine + Glycerin (34%) | 9,15 | | | | | | | | |
| Decyl Glucoside (53%) | | 2,81 | | | 2,81 | 2,81 | | | |
| Aminomethyl Propanol (95%) | | 0,57 | 0,58 | | | | | | 0,34 |
| Coco-Glucoside (52%) | | | 7,69 | 2,26 | | | 7,69 | 7,69 | 2,02 |
| Parfum | | 0,15 | 0,15 | 0,15 | 0,15 | 0,15 | 0,15 | 0,15 | |
| Coco-Glucoside + Glyceryl Oleate (70%) | | | | 0,91 | | | | | 0,79 |
| Disodium Cocoyl Glutamate (25%) | 2,76 | | | | | | | | |
| Acrylic Acid/VP Crosspolymer | 1,10 | 1,00 | 1,20 | 0,90 | 0,90 | 0,80 | 1,10 | 1,00 | 0,90 |
| Ammonium Lauryl Sulfate (30%) | 4,57 | | | | | | | | |
| Sucrose | 64,00 | 64,00 | 64,00 | 64,00 | 64,00 | 64,00 | 64,00 | 64,00 | 64,00 |
| Parfum | | | | | | | | | 0,30 |
| Sodium Coco-Sulfate (92,5%) | | | | 4,11 | | | | | 3,68 |
| **Summen:** | **100,00** | **100,00** | **100,00** | **100,00** | **100,00** | **100,00** | **100,00** | **100,00** | **100,00** |

Die Werte in Klammern hinter den Substanznamen beziehen sich auf die Aktivgehalte.

**Zweite Serie an Beispielen:**

| **Ansatznummer** | **1** | **2** | **3** | **4** | **5** | **6** |
|---|---|---|---|---|---|---|
| **INCI** | **m [%]** | **m [%]** | **m [%]** | **m [%]** | **m [%]** | **m [%]** |
| Aqua | 14,27 | 14,13 | 6,41 | 16,99 | 9,41 | 12,49 |
| Sodium Laureth Sulfate (70%) | 5,4 | 5,61 | - | 6,86 | - | 5,71 |
| Cocamidopropyl Betaine + Glycerin (34%) | 9,41 | 8 | 3,79 | - | - | - |
| PEG-7 Glyceryl Cocoate | 1,02 | 1,06 | - | - | - | - |
| Disodium Cocoyl Glutamate (25%) | - | 1,2 | - | - | - | - |
| Coco Glucoside (52%) | - | - | 4,71 | 6,15 | 7,69 | - |
| Sodium Cocoamphoacetate | - | - | 2,92 | - | - | - |
| Ammonium Lauryl Sulfate (30%) | - | - | 11,07 | - | - | - |
| Coco Betaine | - | - | - | - | 12,9 | 12,9 |
| Sodium Chloride | 1,5 | 1,5 | 1,5 | 1,5 | 1,5 | 1,5 |
| Coco-Caprylate/Caprate | 3 | 3 | 4 | 3 | 3 | 2 |
| Sucrose | 64 | 64 | 64 | 64 | 64 | 64 |
| Citric Acid | qs | qs | qs | qs | qs | qs |
| Aminomethyl Propanol (95%) | qs | qs | qs | qs | qs | qs |
| Acrylic Acid/Vp Crosspolymer | 0,9 | 1 | 1,1 | 1 | 1 | 0,9 |
| Gesamt | 100 | 100 | 100 | 100 | 100 | 100 |

Die Werte in Klammern hinter den Substanznamen beziehen sich auf die Aktivgehalte.

**Dritte Serie an Beispielen (wird auf der folgenden Seite fortgesetzt):**

| **Ansatznummer** | **1*** | **2*** | **3*** | **4*** | **5*** | **6*** | **7*** | **8*** |
|---|---|---|---|---|---|---|---|---|
| **INCI** | **m [%]** | **m [%]** | **m [%]** | **m [%]** | **m [%]** | **m [%]** | **m [%]** | **m [%]** |
| Aqua | 13,14 | 11,60 | 15,24 | 18,34 | 18,24 | 18,14 | 17,94 | 18,54 |
| Citric Acid | | 0,00 | | | | | | |
| Paraffinum Liquidum | 5,00 | 5,00 | 5,00 | 5,00 | 5,00 | 5,00 | 5,00 | 5,00 |
| Aqua + Sodium Hydroxide (45%) | 0,02 | 0,18 | | | | | | |
| Xanthan Gum | | | | | | | | |
| Sodium Chloride | 1,50 | 1,50 | | | | | | |
| Acrylates/C 10-30 Alkyl Acrylate Crosspolymer | | | | | | | | 0,60 |
| Carbomer | | | | | | | 1,20 | |
| Sodium Myreth Sulfate (71 %) | 7,70 | 8,10 | 7,70 | 7,70 | 7,70 | 7,70 | 7,70 | 7,70 |
| Sodium Laureth Sulfate (70%) | | | | | | | | |
| Laureth-2 (99,85%) | 1,20 | 1,26 | 1,20 | 1,20 | 1,20 | 1,20 | 1,20 | 1,20 |
| Decyl Glucoside (53%) | 2,81 | 2,96 | 2,81 | 2,81 | 2,81 | 2,81 | 2,81 | 2,81 |
| Acrylates/C 10-30 Alkyl Acrylate Crosspolymer | | | 0,90 | 0,80 | 0,90 | 1,00 | | |
| Coco-Glucoside (52%) | | | | | | | | |
| Parfum | 0,15 | 0,15 | 0,15 | 0,15 | 0,15 | 0,15 | 0,15 | 0,15 |
| Ammonium Acryloyldimethyltaurate/VP Copolymer | 1,50 | 1,75 | | | | | | |
| Sucrose | 66,98 | 67,50 | 67,00 | 64,00 | 64,00 | 64,00 | 64,00 | 64,00 |
| **Summen:** | **100,00** | **100,00** | **100,00** | **100,00** | **100,00** | **100,00** | **100,00** | **100,00** |

| | | | | | | | | |
|---|---|---|---|---|---|---|---|---|
| * nicht erfindungsgemäßes Beispiel. | | | | | | | | |

Die Werte in Klammern hinter den Substanznamen beziehen sich auf die Aktivgehalte.

**Dritte Serie an Beispielen (Fortsetzung der vorangehenden Seite):**

| **Ansatznummer** | **9*** | **10*** | **11*** | **12*** | **13*** | **14*** | **15*** | **16*** |
|---|---|---|---|---|---|---|---|---|
| **INCI** | **m [%]** | **m [%]** | **m [%]** | **m [%]** | **m [%]** | **m [%]** | **m [%]** | **m [%]** |
| Aqua | 18,44 | 18,34 | 18,54 | 18,44 | 18,34 | 18,24 | 18,14 | 16,45 |
| Citric Acid | | | | | | | | |
| Paraffinum Liquidum | 5,00 | 5,00 | 5,00 | 5,00 | 5,00 | 5,00 | 5,00 | 5,00 |
| Aqua + Sodium Hydroxide (45%) | | | | | | | | |
| Xanthan Gum | | | 0,60 | 0,70 | 0,80 | 0,90 | 1,00 | |
| Sodium Chloride | | | | | | | | |
| Acrylates/C 10-30 Alkyl Acrylate Crosspolymer | 0,70 | 0,80 | | | | | | |
| Carbomer | | | | | | | | |
| Sodium Myreth Sulfate (71%) | 7,70 | 7,70 | 7,70 | 7,70 | 7,70 | 7,70 | 7,70 | |
| Sodium Laureth Sulfate (70%) | | | | | | | | 5,71 |
| Laureth-2 (99,85%) | 1,20 | 1,20 | 1,20 | 1,20 | 1,20 | 1,20 | 1,20 | |
| Decyl Glucoside (53%) | 2,81 | 2,81 | 2,81 | 2,81 | 2,81 | 2,81 | 2,81 | |
| Acrylates/C 10-30 Alkyl Acrylate Crosspolymer | | | | | | | | 1,00 |
| Coco-Glucoside (52%) | | | | | | | | 7,69 |
| Parfum | 0,15 | 0,15 | 0,15 | 0,15 | 0,15 | 0,15 | 0,15 | 0,15 |
| Ammonium Acryloyldimethyltaurate/V P Copolymer | | | | | | | | |
| Sucrose | 64,00 | 64,00 | 64,00 | 64,00 | 64,00 | 64,00 | 64,00 | 64,00 |
| **Summen:** | **100,0 0** | **100,0 0** | **100,0 0** | **100,0 0** | **100,0 0** | **100,0 0** | **100,0 0** | **100,0 0** |

| | | | | | | | | |
|---|---|---|---|---|---|---|---|---|
| * nicht erfindungsgemäßes Beispiel. | | | | | | | | |

Die Werte in Klammern hinter den Substanznamen beziehen sich auf die Aktivgehalte.

**Vierte Serie an Beispielen:**

| **Ansatznummer** | **1*** | **2*** | **3*** |
|---|---|---|---|
| **INCI** | **m [%]** | **m [%]** | **m [%]** |
| Aqua | 21,35 | 12,67 | 13,40 |
| Sodium Chloride | 50,00 | 60,00 | 60,00 |
| Lauryl Glucoside (51,5%) | | 5,83 | |
| Coco-Caprylate/Caprate | 3,00 | 5,00 | 3,00 |
| Cocamidopropyl Betaine + Glycerin (34%) | | 7,35 | |
| Aminomethyl Propanol (95%) | 0,28 | 0,30 | 0,23 |
| Coco-Glucoside (52%) | 2,02 | | 2,02 |
| Coco-Glucoside + Glyceryl Oleate (70%) | 0,79 | | 0,79 |
| Maris Sal | 10,00 | | |
| Disodium Cocoyl Glutamate (25%) | | | 8,00 |
| Acrylic Acid/VP Crosspolymer | 0,50 | 0,50 | 0,50 |
| Ammonium Lauryl Sulfate (30%) | | 8,35 | |
| Parfum | 0,50 | | 0,50 |
| Coco-Sulfate (30% in H2O) | 11,56 | | 11,56 |
| | | | |
| **Summen:** | **100,00** | **100,00** | **100,00** |

| | | | |
|---|---|---|---|
| * nicht erfindungsgemäßes Beispiel. | | | |

Die Werte in Klammern hinter den Substanznamen beziehen sich auf die Aktivgehalte.

## Patentansprüche

1. Kosmetische Reinigungszubereitungen enthaltend Tenside, wasserlösliche Bestandteile in gelöster und kristalliner Form,
wobei die kristalline Form homogen und stabil in der Zubereitung verteilt ist,
wobei es sich bei den wasserlöslichen Bestandteilen um Kohlenhydrate handelt, wobei die Kohlenhydrate mit einem Gehalt von 40 bis 90 Gew.-%, bevorzugt 50 bis 80 Gew.-%, insbesondere bevorzugt 60 bis 75 Gew.-%, bezogen auf das Gesamtgewicht der Zubereitung vorhanden sind,
wobei es sich bei den Kohlenhydraten um Disaccharide, insbesondere Saccharose, handelt,
wobei ein polymerer Strukturant, ausgewählt aus der Gruppe Copolymer aus Vinylpyrrolidon und Acrylsäure vorhanden ist und
wobei die Zubereitungen eine dynamische Viskosität < 80.000mPa·s aufweisen, gemessen mit dem Rheomat R123, Messkörper 2 bei 25°C.

2. Kosmetische Reinigungszubereitungen nach Anspruch 1, **dadurch gekennzeichnet, dass** kein strukturiertes Tensidsystem enthalten ist.

3. Kosmetische Reinigungszubereitungen nach wenigstens einem der vorangehenden Ansprüche, **dadurch gekennzeichnet, dass** der Gehalt an polymeren Strukturanten 0,05 bis 3,0 % Gew.-%, bevorzugt 0,1 bis 2,0 Gew.-% beträgt, bezogen auf das Gesamtgewicht der Zubereitung.

4. Kosmetische Reinigungszubereitungen nach einem der vorangehenden Patentansprüche, **dadurch gekennzeichnet, dass** als Tenside anionische und/oder amphotere und/oder kationische und/oder nichtionische Tenside enthalten sind.

5. Kosmetische Reinigungszubereitungen nach einem der vorangehenden Ansprüche, **dadurch gekennzeichnet**, das der Tensidgehalt 0,1 bis 10 Gew.-%, bevorzugt 0,5 bis 8,0 Gew.-% beträgt, bezogen auf den Aktivgehalt und das Gesamtgewicht der Zubereitung.

6. Kosmetische Reinigungszubereitungen nach einem der vorangehenden Ansprüche, **dadurch gekennzeichnet, dass** der Tensidgehalt > 10 bis 15 Gew.-% beträgt, bezogen auf den Aktivgehalt und das Gesamtgewicht der Zubereitung, wobei die Reinigungszubereitungen kein strukturiertes Tensidsystem aufweisen.

7. Kosmetische Reinigungszubereitung nach einem der vorangehenden Ansprüche, **dadurch gekennzeichnet, dass** Xanthangummi mit einem Gehalt vom 0,05 bis 0,8 Gew.-%, bevorzugt 0,1 bis 0,5 Gew.-% enthalten ist.

8. Verwendung von polymeren Strukturanten, ausgewählt aus der Gruppe Copolymer aus Vinylpyrrolidon zur Hemmung der Sedimentationsneigung von Kohlenhydratkristallen in Kohlenhydrat-gesättigten, tensidhaltigen kosmetischen Reinigungszubereitungen, wobei die Kohlenhydrate mit einem Gehalt von 40 bis 90 Gew.-%, bevorzugt 50 bis 80 Gew.-%, insbesondere bevorzugt 60 bis 75 Gew.-%, bezogen auf das Gesamtgewicht der Zubereitung, vorhanden sind,
wobei es sich bei den Kohlenhydraten um Disaccharide, insbesondere Saccharose, handelt.

## Claims

1. Cosmetic cleansing preparations comprising surfactants, water-soluble constituents in dissolved and crystalline form,
wherein the crystalline form is distributed homogeneously and stably in the preparation,
wherein the water-soluble constituents are carbohydrates,
wherein the carbohydrates are present at a content of 40 to 90% by weight, preferably 50 to 80% by weight, especially preferably 60 to 75% by weight, based on the total weight of the preparation,
wherein the carbohydrates are disaccharides, especially sucrose,
wherein a polymeric structurant is present selected from the group of copolymers of vinylpyrrolidone and acrylic acid and
wherein the preparations have a dynamic viscosity < 80 000 mPa·s, measured with the Rheomat R123, measuring bob 2 at 25°C.

2. Cosmetic cleansing preparations according to Claim 1, **characterized in that** no structured surfactant system is present.

3. Cosmetic cleansing preparations according to at least one of the preceding claims, **characterized in that** the content of polymeric structurants is from 0.05 to 3.0% by weight, preferably 0.1 to 2.0% by weight, based on the total weight of the preparation.

4. Cosmetic cleansing preparations according to any of the preceding claims, **characterized in that** the surfactants present are anionic and/or amphoteric and/or cationic and/or non-ionic surfactants.

5. Cosmetic cleansing preparations according to any of the preceding claims, **characterized in that** the surfactant content is 0.1 to 10% by weight, preferably 0.5 to 8.0% by weight, based on the active content and the total weight of the preparation.

6. Cosmetic cleansing preparations according to any of the preceding claims, **characterized in that** the surfactant content is > 10 to 15% by weight, based on the active content and the total weight of the preparation, wherein the cleansing preparations have no structured surfactant system.

7. Cosmetic cleansing preparation according to any of the preceding claims, **characterized in that**
xanthan gum is present at a content of 0.05 to 0.8% by weight, preferably 0.1 to 0.5% by weight.

8. Use of polymeric structurants selected from the group of copolymers of vinylpyrrolidone for inhibiting the tendency of carbohydrate crystals to sediment in carbohydrate-saturated surfactant-containing cosmetic cleansing preparations,
wherein the carbohydrates are present at a content of 40 to 90% by weight, preferably 50 to 80% by weight, especially preferably 60 to 75% by weight, based on the total weight of the preparation,
wherein the carbohydrates are disaccharides, especially sucrose.

## Revendications

1. Préparations cosmétiques de nettoyage contenant des tensioactifs, des constituants solubles dans l'eau sous forme dissoute et cristalline,
la forme cristalline étant répartie de manière homogène et stable dans la préparation, les constituants solubles dans l'eau consistant en des hydrates de carbone,
les hydrates de carbone étant présents en une teneur de 40 à 90 % en poids, de préférence de 50 à 80 % en poids, de manière particulièrement préférée de 60 à 75 % en poids, par rapport au poids total de la préparation,
les hydrates de carbone consistant en des disaccharides, notamment le saccharose, un structurant polymère choisi dans le groupe des copolymères de vinylpyrrolidone et d'acide acrylique étant présent, et
les préparations présentant une viscosité dynamique < 80 000 mPa·s, mesurée avec Rheomat R123, corps de mesure 2 à 25 °C.

2. Préparations cosmétiques de nettoyage selon la revendication 1, **caractérisées en ce qu'**aucun système tensioactif structuré n'est contenu.

3. Préparations cosmétiques de nettoyage selon au moins l'une quelconque des revendications précédentes, **caractérisées en ce que** la teneur en structurants polymères est de 0,05 à 3,0 % en poids, de préférence de 0,1 à 2,0 % en poids, par rapport au poids total de la préparation.

4. Préparations cosmétiques de nettoyage selon l'une quelconque des revendications précédentes, **caractérisées en ce que** des tensioactifs anioniques et/ou amphotères et/ou cationiques et/ou non ioniques sont contenus en tant que tensioactifs.

5. Préparations cosmétiques de nettoyage selon l'une quelconque des revendications précédentes, **caractérisées en ce que** la teneur en tensioactifs est de 0,1 à 10 % en poids, de préférence de 0,5 à 8,0 % en poids, par rapport à la teneur en agents actifs et au poids total de la préparation.

6. Préparations cosmétiques de nettoyage selon l'une quelconque des revendications précédentes, **caractérisées en ce que** la teneur en tensioactifs est de > 10 à 15 % en poids, par rapport à la teneur en agents actifs et au poids total de la préparation, les préparations de nettoyage ne comprenant pas de système tensioactif structuré.

7. Préparations cosmétiques de nettoyage selon l'une quelconque des revendications précédentes, **caractérisées en ce que** de la gomme xanthane est contenue en une teneur de 0,05 à 0,8 % en poids, de préférence de 0,1 à 0,5 % en poids.

8. Utilisation de structurants polymères, choisis dans le groupe des copolymères de vinylpyrrolidone, pour inhiber la tendance à la sédimentation de cristaux d'hydrates de carbone dans des préparations cosmétiques de nettoyage contenant des tensioactifs, saturées en hydrates de carbone, les hydrates de carbone étant présents en une teneur de 40 à 90 % en poids, de préférence de 50 à 80 % en poids, de manière particulièrement préférée de 60 à 75 % en poids, par rapport au poids total de la préparation, les hydrates de carbone consistant en des disaccharides, notamment le saccharose.
